# EUROPEAN PATENT SPECIFICATION

(11) **EP 0 449 973 B1**
(45) Date of publication and mention of the grant of the patent: **20.03.1996**
(21) Application number: 90901461.5
(22) Date of filing: 20.12.1989
(51) Int. Cl.: A61K 38/00, A61K 47/48, C07K 7/04, C07K 17/02

(54) **POLYPEPTIDE-POLYMER CONJUGATES ACTIVE IN WOUND HEALING**
POLYPEPTID-POLYMER-KONJUGATE MIT WUNDHEILENDER WIRKUNG
CONJUGUES DE POLYPEPTIDE-POLYMERE POUR LE TRAITEMENT DE PLAIES

(30) Priority: 20.12.1988 US 286973
(43) Date of publication of application: 09.10.1991
(73) Proprietor: LA JOLLA CANCER RESEARCH FOUNDATION, La Jolla, CA 92037 (US)
(72) Inventor: PIERSCHBACHER, Michael, D., San Diego, CA 92107 (US); POLAREK, James, W., Del Mar, CA 92014 (US); PICKETT, Marianne, P., San Diego, CA 92109 (US); RUOSLAHTI, Erkki, I., Rancho Santa Fe, CA 92067 (US)
(74) Representative: UEXKÜLL & STOLBERG
(86) International application number: PCT/US89/05771
(87) International publication number: WO 90/06767

(56) References cited:
- WO-A-88/03810
- US-A- 4 578 079
- US-A- 4 703 108
- Dialog Informational Services, File 351, World Patent Index 66-90, WPI Acc. no.88-351056/49; & JP-A-63 264 069
- Dialog Informational Services, File 351, World Patent Index 66/90, WPI Acc. no.89-374110/51; & JP-A-1 279 836

## Description

The present invention relates to peptides conjugated to biodegradable polymers and methods for using the conjugates to promote the healing of wounds in mammals, including humans.

### BACKGROUND OF THE INVENTION

The slow healing or lack of healing of both dermal wounds, such as decubitus ulcers, severe burns and diabetic ulcers and eye lesions, such as a dry eye and corneal ulcer, is a serious medical problem, affecting millions of individuals and causing severe pain or death in many patients. Even the healing of surgical wounds is a problem, particularly in aging and diabetic individuals. Although wounds may be quite dissimilar in terms of cause, morphology and tissue affected, they share a common healing mechanism. Each repair process ultimately requires that the correct type of cell migrate into the wound in sufficient numbers to have an effect: macrophages to debride wounds, fibroblasts for the formation of new collagen and other extracellular matrix components in wounds where the extracellular matrix was damaged, capillary endothelial cells to provide the blood supply and epithelial cells to ultimately cover the wound.

The unwounded dermis owes much of its structure and strength to the interactions of cells with the extracellular matrix. This matrix contains several proteins known to support the attachment of a wide variety of cells. These proteins include fibronectin, vitronectin, thrombospondin, collagens, and laminin. Although fibronectin is found at relatively low concentrations in unwounded skin, plasma fibronectin deposition occurs soon after wounding. When tissue is damaged, the extracellular matrix must be replaced to provide a scaffold to support cell attachment and migration.

In addition to providing a scaffold, extracellular matrices can also direct cellular proliferation and differentiation. An extracellular matrix can, therefore, direct healing of a tissue in such a way that the correct tissue geometry is restored. When applied to wounds, exogenous fibronectin results in increased wound healing, epithelial migration and collagen deposition. However, a number of considerations, including cost, availability and instability, make fibronectin or other extra-cellular matrix proteins less than ideal for such treatment. Moreover, being blood derived products, extracellular matrix proteins may be vectors for infectious disease.

In addition to treatment with fibronectin, attempts have also been made to promote healing by providing cell growth factors such as fibroblast growth factor (FGF) or epidermal growth factor (EGF). Such factors will undoubtedly become useful in treating wounds, but because they cannot effect the correct geometry of the new tissue, they can lead to an overly vascularized tissue and abnormal healing.

It is now recognized that the binding domain of fibronectin and the other adhesion proteins is localized in the amino acid sequence Arg-Gly-Asp-X (also termed RGD-X, in accordance with the standard one letter abbreviations for the amino acids) wherein X can be various amino acids or substituents such that the peptide has cell adhesion promoting activity. Arg-Gly-Asp containing peptides and their uses are disclosed, for example in the following issued United States patents and pending patent applications: Patent numbers 4,578,079 and 4,614,517; Serial numbers 744,981 and 738,078. From these discoveries, work has progressed in the generation of various Arg-Gly-Asp containing peptides having various specificities for particular cell surfaces receptors.

There thus exists a need for an effective agent to promote cell migration and attachment to the site of a wound. Preferably, such an agent should be inexpensive, sterile and have long lasting stability and effectiveness. The present invention satisfies these needs and provides related advantages as well.

### SUMMARY OF THE INVENTION

The present invention provides Arg-Gly-Asp containing peptides and peptide-polymer conjugates that are useful in promoting rapid healing of wounds and methods for preparing a synthetic extracellular replacement matrix (peptide-polymer conjugates) to treat wounds. In one embodiment, the conjugate comprises a peptide containing the amino acid sequence Y-Gly-Asp wherein Y is Arg or D-Arg attached to a biodegradable polymer selected from the group consisting of hyaluronic acid, chondroitin sulfate, heparin, heparan sulfate, polylactase, and polyglycolic acid.

In another aspect, a method of conjugating the peptide to biodegradable polymers is provided. In a further aspect, the invention comprises methods for preparing a synthetic extracellular replacement matrix comprising Y-Gly-Asp containing peptide conjugates for treating wounds. The synthetic matrices according to the present invention are used for the preparation of a pharmaceutical composition for the treatment of wounds.

### BRIEF DESCRIPTION OF THE DRAWINGS

Figure 1 shows the wound strength gains made using the conjugate hyaluronate and R₆GDSPK over a ten day test as compared with the control.

Figure 2 shows the results of Figure 1 as a percentage of the control.

Figure 3 shows the results of the second wound strength experiment using conjugate of hyaluronate-G(dR)₅G₃(dR)GDSPASSK.

Figure 4 shows the data of Figure 3 as a percentage of the control wounds.

### DETAILED DESCRIPTION OF THE INVENTION

The extracellular matrix of mammalian dermis contains several proteins known to support the attachment, and promote the migration and differentiation of a side variety of cells, including fibronectin, vitronectin, collagens and laminin. When the dermis is cut, burned or abraded, the extracellular matrix may be separated or lost. This matrix must be replaced before the wound can be repaired by cellular migration and replication because cells require the matrix to be in place before cell migration, and healing, can occur.

In natural wound healing, the tissue is replaced through the migration of cells and the synthesis of extracellular matrix by them. This process is time-consuming, often requiring more than one year to complete, and results in scarring and in a tissue which is weaker than the surrounding, unwounded tissue.

The present invention provides stable "synthetic matrices" that can be used to support the healing of many different wounds by providing the cells with an attachment base for cell migration.

The "synthetic matrix" comprises a conjugate of a biodegradable polymer and a peptide sequence that dermal cells can use as a binding site during migration. Preferably, the peptide contains the sequence Arg-Gly-Asp or D-Arg-Gly-Asp.

According to the present invention a synthetic extracellular replacement matrix for wound healing is provided, comprising: (a) a synthetic peptide containing the amino acid sequence Y-Gly-Asp, wherein Y is R or dR, said peptide having cell attachment promoting activity; and (b) a biodegradable polymer bonded to said peptide without substantially reducing said affinity for cell binding, wherein said polymer is selected from the group consisting of hyaluronic acid, chondroitin sulfate, heparin, heparan sulfate, polyacetate and polyglycolic acid; wherein said matrix promotes all attachment and migration therein and is not a solid support.

For dermal wounds, these two components will preferably be in the form or a viscous semi-gel, comprising the peptide coupled to a hydrated matrix. The semi-gel is placed directly in the wound at the site or sites of matrix destruction. The wound is otherwise treated in a normal manner. As a semi-gel, the conjugate does not tend to migrate away from the wound site, either due to physical effects such as movement by the patient or by absorption by the patient's own system. The conjugate acts as a temporary replacement matrix that encourages cell migration into the wound and speeds healing. As the wound heals, the conjugate is slowly broken down by the migrating cells and replaced by natural replacement matrix, as evidenced by Example VI. For other applications, such as with corneal abrasion caused by the dry eye condition or other circumstances, a conjugate consisting of an Y-Gly-Asp peptide coupled to chondroitin sulfate is preferable. This conjugate binds to expose dermis collagen matrix, proving attachment sites for corneal epithelial cells. Such material can be provided in liquid form, such as eye drops.

The following standard abbreviations are used herein to identify amino acid residues.

| Amino Acid | Three-letter Abbreviation | One-letter Symbol |
|---|---|---|
| Alanine | Ala | A |
| Arginine | Arg | R |
| D-Arganine | D-Arg | dR |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| nMe refers to an n-Methyl group. | | |

A portion of the preferred amino acid sequences of the present invention is derived from that of fibronectin. Fibronectin is a naturally occurring protein that is present in low levels in undamaged dermis and in higher levels in damaged dermis. Fibronectin has been shown to be an important factor in healing, because it provides a binding site for migrating cells coming in to replace cells lost due to injury. The amino acid sequence in fibronectin required for cell bonding is Arg-Gly-Asp-X where X is serine. However, X can be other amino acids or substituents such that the peptide has cell-attachment promoting activity. (Pierschbacher and Ruoslahti, Proc. Natl. Acad. Sci. USA 81:5985-5988 (1984)). Arg-Gly-Asp is also a cell attachment promoting sequence in a number of other adhesive proteins including vitronectin, collagen, fibrin and tenascin. Synthetic peptides containing the sequence are capable of promoting cell attachment when they are presented as an insoluble substrate as well as inhibiting cell attachment to fibronectin or other adhesive proteins when in solution. Several receptors for this peptide sequence have been identified in various cell types (Pytela et al., Cell 40:191-198 (1985); Ruoslahti and Pierschbacher, Science 238:491-497 (1987)). Depending on the type of cell to be attached, the Arg-Gly-Asp-X sequences may be modified to bind preferentially to individual receptors and, therefore, can attract individual types of cells (Pierschbacher and Ruoslahti, J. Biol. Chem. 262:14080-14085 (1987)). In addition, most extracellular matrix proteins contain a basic "heparin-binding" region which appears to enhance all adhesion. This quality can be mimicked by polyarginine, polylysine or polyornithine, or by other basic residues. Moreover, the peptide can be linear or cyclic. It is intended that claims hereto include all such additions and substituents.

Accordingly, in the preferred embodiments of the invention, polypeptides that are useful in combination with a biodegradable polymer to promote wound healing include the following: 
R R R R R R G D S P K;

G (dR) G D S P A S S K;

nMe G (dR) (dR) (dR) (dR) (dR) G G G
(dR) G D S P A S S K;

G (dR) (dR) (dR) (dR) (dR) G G G (dR)
G D S P A S S K; and

G (dR) (dR) (dR) (dR) (dR) G G G (dR)
G D S P A S S K; and
wherein R is the amino acid Arginine (Arg); dR is the amino acid D-Arginine (D-Arg); G is the amino acid Glycine (Gly); D is the amino acid Aspartic acid (Asp); S is the amino acid Serine (Ser); P is the amino acid Proline (Pro); K is the amino acid Lysine (Lys); A is the amino acid Alanine (Ala); and nMe is an n-methyl group.

These peptides are synthetic, relatively easy to manufacture (as compared, for example, to fibronectin) and do not need to be extracted from blood. In addition, the smaller size of the peptide (as compared, for example, to fibronectin) allots many more binding sites to be attached to a given volume of biodegradable polymer. These peptides are also much more stable than fibronectin in solution. In particular, D-Arg confers protease resistance. Moreover, because the peptides do not carry species-specific immunological determinants, they can therefore be used in both veterinary and human applications. Preferably such peptides contain at least one group of at least three amino acids selected from the D or L forms of Arg, Lys, or ornithine to the N terminal side of the Arg-Gly-Asp sequence.

The biodegradable polymer is selected from the group consisting of hyaluronic acid, chondroitin sulfate, heparin, heparan sulfate, polyacetate and polyglycolic acid.

A preferred biodegradable polymer of the invention is hyaluronic acid. Hyaluronic acid consists of alternating residues of D-glucuronic acid and N-acetyl-D-glucosamine. The polymer is found in the dermal matrix and is commercially available from many sources in pure form. It can form gels or viscous solutions because it is a water soluble polymer. The polymers may be cross-linked to stabilize their physical properties.

An alternatively preferred biodegradable polymer, particularly for ophthalmic applications due to its ability to specifically bind exposed collagen/matrix, is chondroitin sulfate. Such conjugates form stable solutions which can be provided in liquid form, such as eye drops. Other polymers that can be advantageously used include heparin, heparan sulfate, dextran, polylactate and polyglycolic acid. In addition, these materials could be used in dermal application by crosslinking them to form a gel, or by forming mesh-like structures.

The two components of the wound healing conjugate can be joined by a number of procedures. Preferably, they are joined using 1-ethyl-3- (3-dimethylaminopropyl) carbodiimide (EDC), a cross-linking/coupling agent. Other cross-linking/coupling reagents can also be used such as dicyclohexylcarbomide (DDC), glutaraldehyde, cyanogen bromide or N-hydroxysuccinimide. Other methods, well known in the art, can alternatively be used.

A peptide-hyaluronic acid conjugate forms a semi-gel the viscosity of which can be altered by the addition of unconjugated hyaluronate or varying the degree of peptide conjugate. Preferably the ratio of peptide to the polymer is 0.2 - 0.3:1, although other ranges are also useable. The semi-gel can be placed directly in a wound to aid in healing by providing an artificial biodegradable matrix. Growth factors, such as transforming growth factor β (TGF-β) or platelet-derived growth factor (PDGF) may be used along with the conjugate to further promote healing. In alternate embodiments, the conjugate can be coated on a biodegradable mesh or other implanted material, or can itself be formed into sheets or other structures, or can be provided as a stable solution. Such conjugates can be used on any wounds which involve body tissues being cut, abraded or otherwise damaged. Such wounds include chronic skin ulcers, burns, corneal wounds and incisions. Regeneration of tissue (such as cartilage, bone, or nervous tissue) can also be enhanced by applying the conjugate.

The following examples are intended to more clearly illustrate aspects of the invention, but are not intended to limit the scope thereof.

### EXAMPLE I

### PEPTIDE SYNTHESIS

The peptide G (dR) (dR) (dR) (dR) (dR) G G G (dR) G D S P A S S K was synthesized using an automated peptide synthesizer (Model 430A; Applied Biosystems, Foster City, California) according to the directions provided by the manufacturer. After cleavage from the resin with hydrogen fluoride, the peptides were washed in cold ethyl ether and precipitated from solution in trifluoroacetate with ice cold ether. The peptides were then redissolved in distilled water and lyophilized. The peptides were further purified by HPLC using a Waters Bondapak™ C₁₈ (3 X 30 cm; 10 µm packing, Waters Assoc., Milford, MA). Other peptides can be made by the same method.

Alternatively, for larger volumes, the peptide can be prepared by the following method. Approximately 30 grams of t-boc lysine polystyrene resin (containing approximately 19.5 mM of t-boc lysine) was weighed out and placed in a glass peptide synthesis reaction vessel. The weight of the resin can be increased or decreased depending on the desired amount of desired product. The following amino acids with protecting groups as indicated were used for the production of the peptide:
l-Serine (benzyl; bzl)
l-Alanine
l-Proline
l-Aspartic Acid (o-cyclohexyl; OcHx)
Glycine
d-Arginine (p-toluenesulfonyl; tos)
Each amino acid was weighed out in a quantity so as to give a 4-fold ratio on a molar basis with that of the t-boc lysine and was loaded into individual 500 mL glass erlenmeyer flasks.

Each amino acid (excluding the t-boc lysine polystyrene resin) was dissolved and activated by the addition of 1 M hydroxybenzotriazole in N-methylpyrrolidone. Hydroxybenzotriazole was added in a volume to give an equimolar concentration with that of the amino acid. Activation of each amino acid (excluding the t-boc lysine polystyrene resin) was completed by adding a volume of 1 M dicyclohexylcarbodiimide in N-methylpyrrolidone to give an equimolar concentration of dicyclohexylcarbodiimide with that of the dissolved amino acid. The materials were incubated for at least 30 minutes to allow complete activation. Each amino acid was converted to the Hobt active ester. The activated amino acids were filtered through Whatman #1 filter paper to remove any insoluble material.

In order to remove the t-boc protecting group from the amino terminus of the N-terminal amino acid, the t-boc amino acid-polystyrene resin was swelled and then washed three times with dichloromethane (approximately 12 mL per gram of resin) for 5 minutes. After each wash, the liquid was removed from the reaction vessel. After swelling and washing, the resin was acidified by washing with 50% trifluoroacetic acid in dichloromethane (approximately 6 mL per gram of resin) for 2 minutes. The liquid was removed from the reaction vessel. Deprotection of the t-boc amino acid-polystyrene resin was completed by adding an additional volume of 50% trifluoroacetic acid in dichloromethane (approximately 12 mL per gram of resin) and allowing the material to incubate for approximately 30 minutes or longer.

After deprotection, the trifluoroacetic acid in dichloromethane was removed from the reaction vessel and the deprotected amino acid-polystyrene resin was washed two times with dichloromethane (approximately 12 mL per gram of resin). The deprotected amino acid-polystyrene resin was washed two times with dichloromethane isopropanol wash (50% v/v, approximately 12 mL per gram of resin) and the washes discarded. The resin was then washed two times with dichloromethane (approximately 12 mL per gram of resin).

The acidified, deprotected amino acid-polystyrene resin was neutralized by washing three times with 10 mM diisopropylethylamine in dichloromethane (approximately 12 mL per gram of resin). The neutralization washes were discarded after each cycle. The neutralized amino acid polystyrene resin was washed three times with dichloromethane (approximately 12 mL per gram of resin) and three times with N-methylpyrrolidone (approximately 12 mL per gram of resin). The washes were discarded after each cycle.

The appropriate, activated amino acid was added to the reaction vessel containing the amino acid-polystyrene resin in a quantity to give a molar ratio of 4 to 1 (activated amino acid to deprotected amino acid linked to resin). The appropriate amino acid was selected according to the desired peptide sequence. The material was mixed for 30 minutes or longer to allow the coupling reaction to go to completion. The reaction was monitored using ninhydrin reactivity. The resin containing the coupled amino acids was washed two times with N-methylpyrrolidone (approximately 12 mL per gram of resin) and two times with dichloromethane (approximately 12 mL per gram resin). Each wash was discarded at the completion of the cycle.

The process beginning at acidification and deprotection of the t-boc amino acid polystyrene resin through this step was repeated for each subsequent amino acid to be added to the peptide chain. The amino acid sequence followed for manufacture of the peptide is listed below:

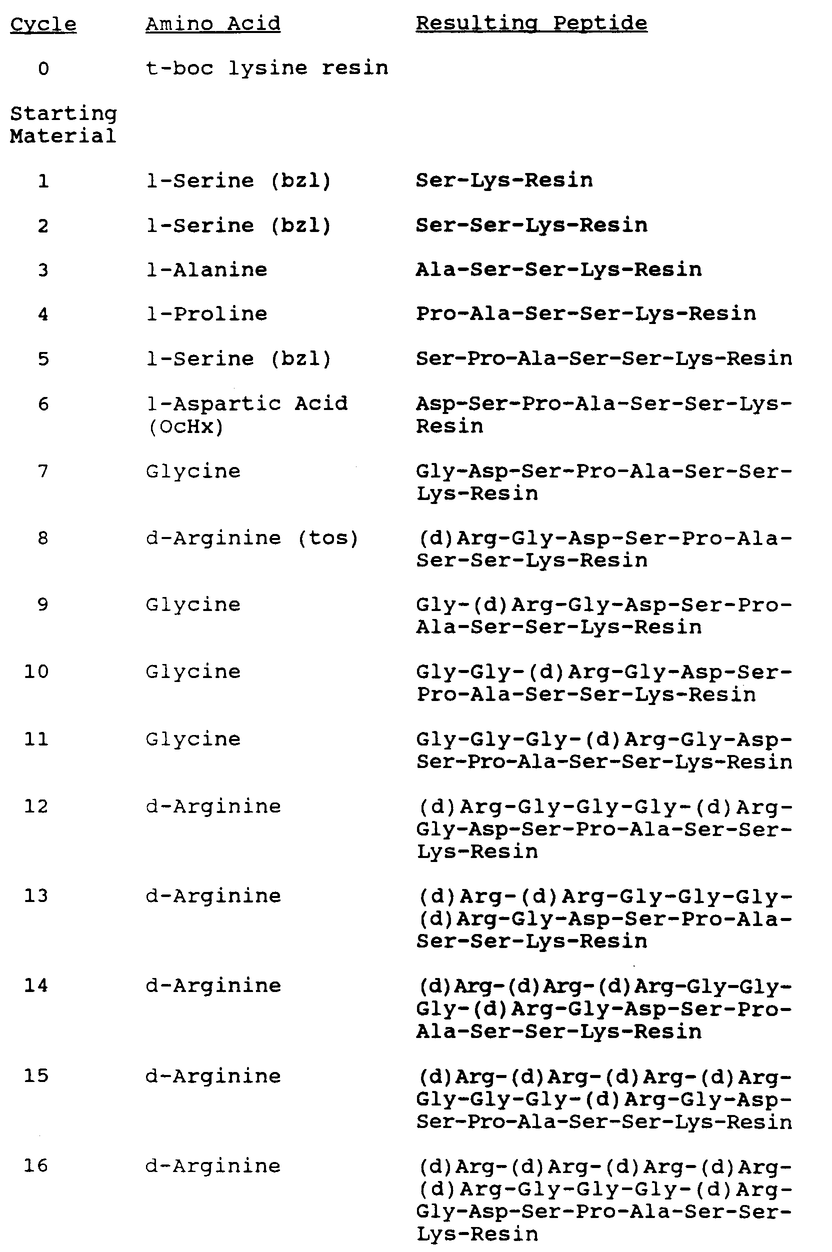

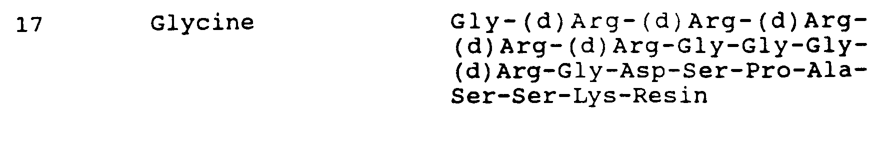

After completion of all coupling steps for the peptide, the resin was washed twice in dichloromethane (approximately 12 mL per gram of resin each wash). The resin was collected and dried by purging with argon gas. The dried resin is desiccated and stored at ambient temperature until further processing.

The dried and weighed resin was placed into an appropriate sized glass reaction vessel containing a stir bar. Anisole was added to the resin at a volume of 1 ml per gram of peptide resin with stirring. Pentamethylbenzene was added to the reaction vessel containing anisole and peptide resin in an amount equal to 50 mg pentamethylbenzene per gram of peptide resin. The contents of the vessel were cooled to approximately -70°C. Then hydrogen fluoride gas was condensed into the reaction vessel at a volume of 10 ml per gram of peptide resin. The temperature of the reaction vessel was raised slowly to approximately -15°C with stirring and was maintained below 0°C at all times for 60 minutes or longer. At the completion of the cleavage step and the removal of protecting groups, all hydrogen fluoride was evaporated from the reaction vessel by purging with nitrogen gas for approximately 1 hour while the contents of the vessel were maintained under stirring conditions with the temperature below 0°C.

The cleaved peptide was solubilized in HPLC-grade water and ethyl ether (anhydrous) in an approximate water to ether ratio of 1:1.5 and filtered through a glass fritted funnel to remove the insoluble resin from the free peptide. After filtration, the aqueous phase containing the cleaved peptide was collected and re-extracted two additional times with ethyl ether (anhydrous). The pH of the soluble aqueous peptide solution was adjusted to 6.5 - 7.5 with 2 M ammonium bicarbonate. The neutralized solution was shell frozen and lyophilized to recover the crude peptide.

Reverse Phase High Performance Liquid Chromatography (RPHPLC) was utilized for the purification of the synthetic peptide. This procedure exploits the hydrophobicity of the peptide sequence. The peptide was loaded onto the column using an aqueous polar solvent to assure column binding. The peptide was effectively purified by exchanging the polar aqueous solvent with that of a non-polar organic solvent using a linear gradient approach.

Approximately 6 grams of crude lyophilized peptide were dissolved in approximately 100 mL HPLC water containing 0.05% TFA (Buffer A) and filtered through Whatman #1 filter paper into a glass flask. The aqueous solution of crude peptide was loaded onto a 30 cm radial compression C-18 column pre-equilibrated with Buffer A at an approximate flow rate of 30 mL per minute. After loading crude peptide, the column was flushed with approximately 50 mL of Buffer A at an approximate flow rate of 30 mL per minute to assure peptide binding to column. Peptide was eluted by increasing the acetonitrile to water gradient from 0 to 20% over a 50 minute period. The purification was followed by monitoring the absorbance at 210 nm. Peptide peaks were collected manually at 12 - 15 minutes and 34 - 38 minutes. This material was approximately 80% pure as determined by peak integration. The semi-purified peptide was shell frozen, lyophilized and stored in a desiccator at room temperature.

The semi-purified peptide was dissolved in Buffer A at an approximate volume of 100 mL per 900 mg of peptide and filtered through Whatman #1 filter paper into an appropriate sized siliconized glass container. The aqueous solution of semi-purified peptide was loaded onto a two inch by 30 cm radial compression, pre-equilibrated column in an amount equivalent to 1 gram of partially purified peptide per 350 grams of C-18 resin at a flow rate of approximately 30 mL per minute. The column contained 350 grams of 15µ C-18 resin. Column size was based on the quantity of peptide to be loaded. For larger scale purifications, a four inch by 60 cm column was used. After loading semi-purified peptide onto the column, the column was flushed with approximately 50 - 100 mL of Buffer A at an approximate flow rate of 30 mL per minute to assure peptide binding to column. Peptide was eluted by increasing the acetonitrile to water gradient from 0 to 7% over a 20 minute period using a flow rate of approximately 30 mL per minute. Isocratic elution at 7% acetonitrile concentration continued for approximately 60 minutes at which time the peptide was eluted. Fractions were collected at 0.3 minute intervals. Pure fractions were pooled and lyophilized after analytical HPLC analysis. Each pool was considered a batch. One percent of each batch of purified peptide was sampled, pooled together and analyzed by the reverse phase C-18 HPLC. Pooled samples must show a purity profile greater than 95% with no impurity greater than 1%. The pure peptide was then coeluted with an in-house standard as an in process control. The pooled peptide batches were dissolved in HPLC grade water and filtered through Whatman #1 filter paper. The yield from the synthesis was calculated. The large mix of pooled peptide was frozen, lyophilized, weighed and stored in a desiccator at room temperature until used in final product manufacture.

The purified peptide was analyzed using quantitative amino acid analysis (Table II), amino-terminal sequence analysis (Table III), and fast atom bombardment mass spectroscopy (FAB-MS). All of the results were consistent with the expected structure of the peptide. The molecular ion from four (FAB-MS) analyses was 1913.2 ± 0.5, very close to the theoretical value of 1913.5.

**TABLE II**

| AMINO-TERMINAL SEQUENCE ANALYSIS OF THE PEPTIDE OF EXAMPLE I | | |
|---|---|---|
| CYCLE | RESIDUE | NANOMOLES |
| 1 | G | 9.0 |
| 2 | R | 2.1 |
| 3 | R | 2.7 |
| 4 | R | 3.2 |
| 5 | R | 3.7 |
| 6 | R | 3.8 |
| 7 | G | 6.1 |
| 8 | G | 6.2 |
| 9 | G | 5.4 |
| 10 | R | 2.1 |
| 11 | G | 3.7 |
| 12 | D | 3.2 |
| 13 | S | 1.9 |
| 14 | P | 1.5 |
| 15 | A | 1.7 |
| 16 | S | 0.9 |
| 17 | S | 0.8 |
| 18 | K | 0.2 |

**TABLE III**

| AMINO ACID ANALYSIS OF THE PEPTIDE OF EXAMPLE I | |
|---|---|
| AMINO ACID | RESIDUES/MOLE (N=6) |
| Asp | 1.01 ± 0.03 |
| Ser | 2.61 ± 0.06 |
| Pro | Detectable |
| Gly | 4.82 ± 0.05 |
| Ala | 1.09 ± 0.06 |
| Lys | 1.02 ± 0.02 |
| Arg | 6.14 ± 0.08 |

### EXAMPLE II

### PREPARATION OF PEPTIDE HYALURONIC ACID CONJUGATE

Conjugates were made by mixing a solution of hyaluronic acid, (bacterially derived; Genzyme, Boston, MA) buffered in phosphate buffered saline, with the coupling agent 1-ethyl-3- (3 dimethylaminopropyl) carbodiimide at a concentration of 0.07 M for 30 minutes. After this time, the peptide of Example I was added at a weight equal to that of the hyaluronate. This mixture was shaken overnight, after which the product was purified by dialysis and precipitation with 3 volumes acetone or ethanol. Typical preparations consisted of a conjugate containing between 20 and 30% peptide by weight. Addition of sterile phosphate buffered saline results in clear solutions, the viscosity of which can be adjusted by the addition of uncoupled hyaluronate.

In order to separate free peptide from conjugate, dialysis was carried out using standard dialysis membranes with a nominal molecular weight cut off of 8 - 14,000. 100 ml of sample (with starting concentration of HA and peptide at 4 mg/ml) was dialyzed against 2 liters phosphate buffered saline, pH 7.4, with buffer changes three times daily, for a total of 50 hours. Peptide was analyzed by standard protein assay (BCA assay, Pierce Chemical, Rockford, IL) and 1-ethyl-3- (3-dimethylaminopropyl) urea (EDU) was assayed using amino acid analysis.

As shown in Table IV, a low molecular weight byproduct (EDU); of the coupling procedure was dialyzed away from the conjugate, while the peptide remains with the conjugate. Note that the conditions shown duplicate the normal coupling procedure. The amount of peptide remaining was 20-30% of the starting material. Since we begin with equal amounts, the peptide was 20-30% of the amount of hyaluronate in the final product.

**TABLE IV**

| DIALYSIS RATE | | |
|---|---|---|
| Time | EDU (mg/ml) | Peptide (mg/ml) |
| 0 | 11.000 | 4.00 |
| 10 | 0.660 | 2.16 |
| 20 | 0.110 | 1.25 |
| 30 | 0.016 | 0.95 |
| 40 | 0.005 | 0.62 |
| 50 | 0.002 | 0.52 |

### EXAMPLE III

### PREPARATION OF A CHONDROITIN SULFATE CONJUGATE

The peptide of Example I was conjugated to chondroitin sulfate by mixing chondroitin sulfate (Hepar Industries, Inc., Franklin, OH) at 100 mg/ml in water with 1-ethyl-3-(3-dimethylaminopropyl) carbodiimide (EDC) at 70 mM for 30 minutes, followed by addition of the peptide of Example I at 10 mg/ml and shaking overnight at room temperature. Conjugates were then precipitated by the addition of NaCl to 1% and 3 volumes of acetone, followed by centrifugation for five minutes at 2000xG. The conjugate was then dried under vacuum and resuspended in phosphate buffered saline to 100 mg/ml. Typical peptide incorporation was 74 mg peptide/g conjugate. Chondroitin sulfate conjugate resulted in attachment at the level of 700 to 1000 cells/mg conjugate, determined as described in Example IV.

### EXAMPLE IV

### PRODUCTION OF PEPTIDE CONJUGATES WITH VARIOUS BIOPOLYMERS

The peptide as prepared in Example I was conjugated to potato starch using the cyanogen bromide activation method as follows: 25 mg CNBr was dissolved in 20 ml H₂O. The pH was raised quickly to pH 11 with 1N NaOH, at which time 500 mg potato starch (Sigma Chem. Co., St. Louis, MO) was added. The pH was maintained at 11 for 3, 6 or 12 minutes (by addition of additional NaOH and with constant stirring) at which time the starch was collected on filter paper (Whatman No. 2) and washed with cold water and acetone. 200 mg of these activated samples were added to 10 ml coupling buffer (0.2 M sodium bicarbonate, pH 9.5) containing 20 mg of the peptide YAVTGRGDSPASSKPISNYRTELLDKPSQM and stirred overnight in the cold. In the morning, conjugates were collected on Whatman #2 filter paper, washed with coupling buffer, 0.01N HCl, 1 M NaCl, and graded water/acetone (80%, 60%, 40%, 20%), then acetone. Peptide incorporation was assayed by ninhydrin assay. Yield was approximately 10 mg peptide incorporated/g starch.

Alternatively, the peptide were conjugated to potato starch using dicyclohexylcarbodiimide (DDC) activation. 0.2 g potato starch was suspended in 6 ml dioxane, 100 µg DDC was added, and the mixture shaken for 30 minutes. The starch was then collected on a fritted glass filter, and washed with dioxane. After air drying, it was added to 8 ml of 1M Na₂CO₃, pH 10. 1.2 mg peptide was added, and the mixture shaken overnight at 4°C. In the morning, conjugates were collected by filtration, washed with water, 1 M NaCl, water and graded acetone/water as before. Peptide incorporation was assayed by ninhydrin. Typical yields were 3 to 4 mg peptide incorporated/g potato starch.

The peptide was also conjugated to agarose (Bethesda Research Laboratories, Gaithersburg, MD) as by the above dicyclohexylcarbodiimide activation method using 40 µg of DDC in place of 100 µg of DDC.

A peptide prepared by the method of Example I was conjugated to Sepharose according to a modification of the method of Pierschbacher and Ruoslahti (Nature 309:30-33 (1984), which is incorporated herein by reference). 50 mg of Sepharose in 7.5 ml 1M Na₂CO₃ was activated with 20 mg CNBr for 10 minutes. After collection and washing, the samples were resuspended in 5 ml 1M Na₂CO₃, and shaken with 1 mg of the peptide GRGDSPK overnight in the cold. The conjugates were then collected and washed as before, and peptide incorporation assayed by BCA protein assay (Pierce Chemical Co.) using peptide as standard. Incorporation was at the level of 5 mg/ml beads.

These conjugates were tested for cell attachment by immobilizing them onto tissue culture plastic coated with 100 µg/ml rabbit immunoglobulin G. This was done through the addition of the conjugate to the well, followed by the addition of concentrated 1-ethyl-3- (3-dimethylaminopropyl) carbodiimide (EDC) to couple the conjugate to the protein coating. After 4 hours, the wells were washed, and cells (normal rat kidney cells at 2 x 10⁵/ml) added and incubated for 1 hour at 37°C. After fixation and staining, cell attachment was assayed.

All conjugates showed peptide-dependent cell attachment; there was little or no cell attachment to the biopolymer alone but significant cell attachment to the peptide-biopolymer conjugate. The potato starch conjugates resulted in binding of approximately 150 cells/mg conjugated starch gel.

### EXAMPLE V

### MEASUREMENT OF WOUND STRENGTH AND HEALING AFTER TREATMENT WITH PEPTIDE-HYALURONATE CONJUGATES

Wistar Furth adult male rats (175-250g) were anesthetized by intraperitoneal injection of pentobarbital (60 mg/kg body weight). The rats dorsal area was closely shaved, and each animal was positioned and secured for surgery. Using aseptic technique, five full thickness skin incisions, 1.5 cm long, were made on the dorsal area. Each incision was treated with 0.05 - 0.10 ml of a blind sample of the peptide RRRRRRGDSPK-conjugated to hyaluronate or with 0.05 -0.10 ml of human fibronectin (3 mg/ml in PBS, Collaborative Research, Bedford, MA) or rat fibronectin (2 mg/ml in PBS, Telios Pharmaceuticals, Inc., San Diego, CA) or was left untreated as a control. After treatment, the incisions were closed using standard 4-0 nylon sutures for placement of 5 to 7 interrupted sutures. The rats were returned to their cages for recovery and observation. At post operative days 2 through 7 and 10, six animals were anesthetized as above and were euthanized by an intracardiac injection of 0.5 cc of T-61.

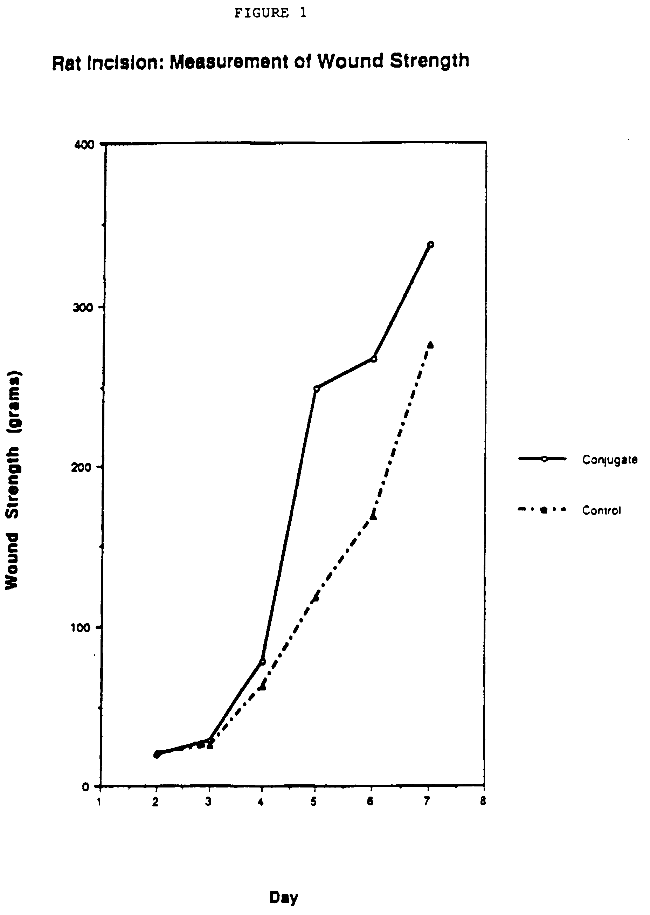

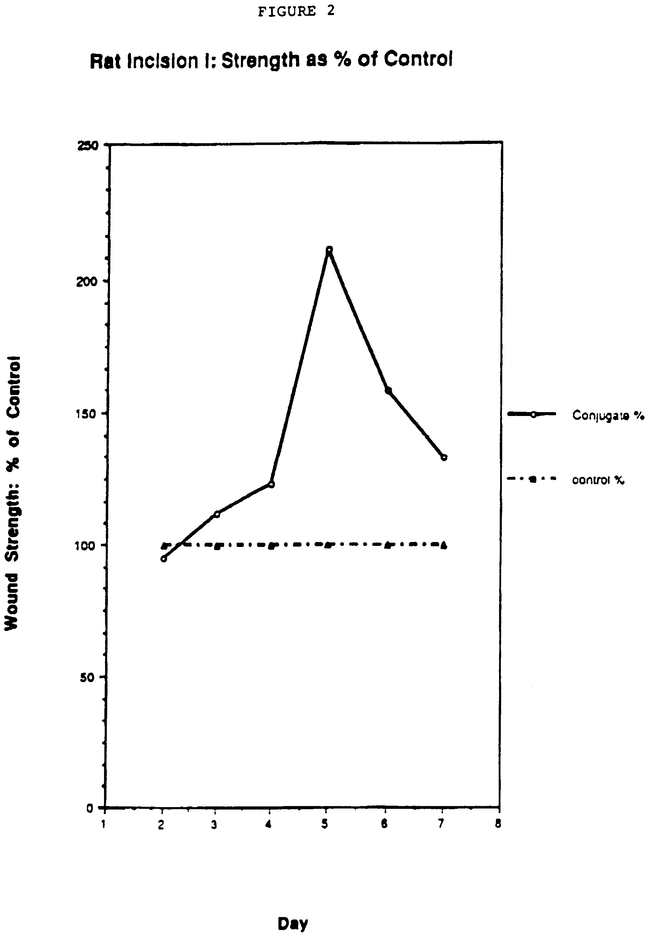

Following euthanasia, the pelt was removed from the rat and 5 mm wide by 2 cm long skin strips bisecting incisions were prepared. Histological specimens from the skin adjacent to the tensile strength strip were also taken to measure tensile strength, one end of the strip was clipped using a 1 mm Michel Clipper (Militex Instruments Co.) which was tied to a Grass model FTO3C force displacement transducer. The other end of the strip was clipped using a light weight hemostat which was attached to a Harvard Model 901 push-pull infusion pump (Harvard Instruments, Millis, Mass.) using cotton thread over a pulley. The transducer was attached to a Grass polygraph (Model 7D) for recording the results. After calibration, the pump was turned on to pull at the rate of 64 mm/min. The amount of force required to pull the skin apart at the incision was recorded on the polygraph. The results are shown in Figures 1 and 2. As these figures show, wound strength was increased by use of the conjugate for days 3 through 7 after incision, with the maximum effect being seen at day 5, where the conjugate-treated wounds achieved 211% of the strength of untreated wounds.

### EXAMPLE VI

### TESTING OF PEPTIDE-HYALURONATE CONJUGATES IN A POLYVINYLALCOHOL SPONGE IMPLANT MODEL

Peptide-hyaluronate conjugates were tested using the model of Davidson, et al., J. Cell Biol. 100:1219-1227 (1985), which is incorporated herein by reference. This model involves the implantation of inert PVA sponges loaded with the conjugates or control materials under the skin of rats. The sponges were removed from the animals at 4 to 10 days after implantation and analyzed for cell ingrowth number and type and as collagen content, blood vessel formation (angiogenesis) and inflammatory response, as well as persistence of the conjugate in the wound. Results of these studies indicated:
A. Sponges containing hyaluronate conjugated with the peptide R₆GDSPK by the method of Example II gave rise to increased angiogenesis, collagen secretion and fibroblast migration at early time points, with the effect diminishing at later time points, such that the sponge appeared similar to the control sponges by the end of the experiment, except that more collagen was continuously deposited into the sponges loaded with hyaluronate-peptide conjugates.
B. Hyaluronate conjugates slowly disappeared from the sponge, with markedly less conjugate present by 14 days after implantation.

### EXAMPLE VII

### TESTING OF PEPTIDE-HYALURONATE CONJUGATES IN RAT DERMAL INCISIONS

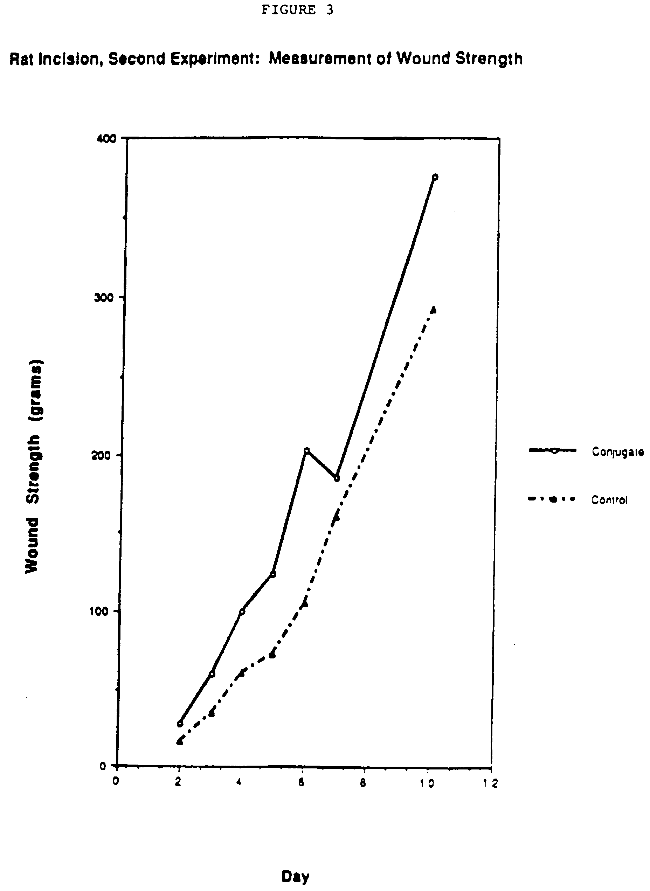

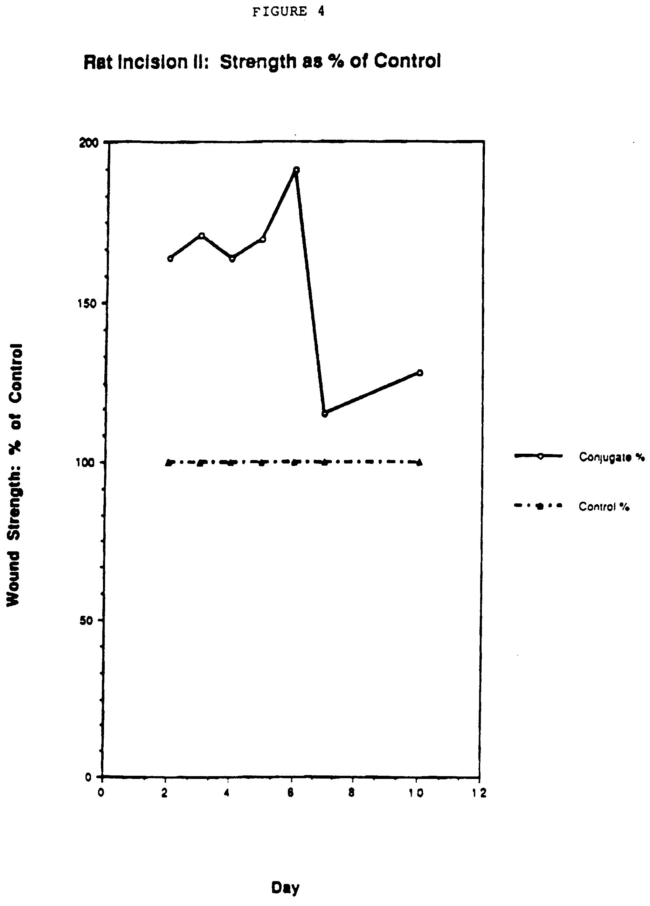

The methods of Example V were used to test whether the conjugates employing the peptide G(dR)₅G₃(dR)GDSPASSK (which was found to have more activity than the RGDSPK peptide conjugate tested in Example V in in vitro assays of the type described in Pierschbacher, et al., Natl. Acad. Sci. 80:1224-1227, (1983)) also had superior activity in vivo. As can be seen in Figures 3 and 4, conjugates of this peptide showed earlier activity (attaining 160% of control on day 2) and persistent activity (maintaining activity greater than control through day 10).

A mixture of hyaluronate and peptide (formed by mixing equal amounts of the hyaluronic acid and peptide) was tested in a wound strength model using the above protocol. At day 4, the point of maximal activity of the conjugate, the activity of the mixture was 100.8% of the control, while that of the conjugate was 125% of the control. As can be seen from the data, the mixture showed no wound healing activity.

### EXAMPLE VIII

### TREATMENT OF DRY EYE SYNDROME WITH CHONDROITIN-SULFATE PEPTIDE CONJUGATES

The dry eye condition was induced in six cats on day zero by excision of the tear ducts and nictitating membranes from the eyes. The cats were divided into four groups and treated as follows:

Group one was treated with two drops of human fibronectin, 3 mg/ml, twice a day from day 17 to day 34, and two drops a day three times a day from day 34 to 43. Group two was treated with two drops of chondroitin sulfate peptide conjugate, G(dR)₅G₃(dR)GDSPASSK 100 mg/ml, twice a day from days 17 to 34, and two drops a day three times a day from day 34 to day 43. Group 3 was treated with phosphate buffered saline, two drops, twice a day Group 4 was treated.

Eyes were examined by impression cytology, in which an impression of the surface of the eye is taken by the placement of a polymer on the eye surface an the cells stuck to the surface examined microscopically, and by staining the eyes with rose bengal, a reddish stain which selectively stains the exposed matrix and underlying basal epithelial cells but does not stain undamaged corneal epithelium.

Cats in Groups one and two, treated with fibronectin or the peptide conjugate, exhibited marked reduction of the dry eye condition. Cats treated with phosphate buffered saline alone and untreated cats did not show any change in eye condition over the time shown.

### EXAMPLE IX

### TREATMENT OF EPIDERMAL WOUNDS

Three young specific pathogen free (SPF) pigs weighing 20 to 25 kg were conditioned for two weeks prior to experimentation. The animals received water and a basal diet without antibiotics (Purina Control Factor) ad libitum and were housed individually in our animal facilities (meeting American Association for Accreditation of Laboratory Animal Care [AAALAC] compliance) with controlled temperature (19°C to 21°C) and light and dark (12h/12h LD).

The experimental animals were clipped with standard animal clippers. The skin on the back and both sides of the animal were prepared for wounding by washing with a non-antibiotic soap (Neutrogena^{R}).

On the day of wounding (Day 0), the pigs were anesthetized with ketamine (I.M.) and inhalation of a halothane, oxygen and nitrous oxide combination. Five specially designed cylindrical brass rods weighing 358 g each were heated in a boiling water bath to 100°C. A rod was removed from the water bath and wiped dry before it was applied to the skin surface to prevent water droplets from creating a steam burn on the skin. The brass rod was held at a vertical position on the skin, with all pressure supplied by gravity, for six seconds, to make a burn wound 8.5 mm diameter x 0.6 mm deep. Immediately after burning, the roof of the burn blister was removed with a sterile spatula. The burn wounds were made approximately 2 cm from each other. Hyaluronic acid alone was used as a control. Peptide hyaluronic acid conjugate was made (bacterially derived; Genzyme) by the method of Example II. Wounds were dressed with or without Tegaderm^{R} (3M; St. Paul, MN) an occlusive dressing which prevents air circulation.

The wounds on three animals were divided into three treatment groups and treated as follows:

| Animal | I | II | III |
|---|---|---|---|
| 1 | air exposed | peptide conjugate/Tegaderm | hyaluronic acid/Tegaderm |
| 2 | air exposed | Tegaderm | peptide conjugate/Tegaderm |
| 3 | air exposed | Tegaderm | peptide conjugate/Tegaderm |

Approximately 110 to 120 burn wounds were made on the anterior two-thirds of the animal. The posterior third of the animal was not used because of anatomical differences, resulting in a more rapid healing of posterior burns.

The burn wounds were treated once with enough material to cover the wound (approximately 0.1 ml) immediately after wounding. The peptide-conjugate and hyaluronate (vehicle) were occluded with Tegaderm dressing. The animals were wrapped with Coban^{R} adhesive bandages (Johnson and Johnson, New Brunswick, NJ) to secure dressings in place. Dressings were kept in place throughout the entire experiment.

On Days 7 through 15, five burn wounds from each treatment group were surgically excised using the electrokeratome (0.6 mm deep). Any specimens that were excised, but not intact, were discarded. The excised burn wounds and the surrounding normal skin were incubated in 0.5 M NaBr for 24 hours at 37°C (Figure 4). After incubation the specimens were separated into epidermal and dermal sheets. The epidermis was then examined macroscopically for defects in the area of the burn wounds. Epithelialization was considered complete if no defect was present (healed); any defect in the burn area indicates that healing is incomplete. The epidermal sheet was placed on cardboard for a permanent record.

The number of wounds healed (epithelialized) was divided by the total number of wounds sampled per day and multiplied by 100, as shown in Table I.

On day 9 post wounding the peptide-hyaluronate conjugate treated wounds were 90% epithelialized as compared to air exposed, hyaluronate and control treated wounds, 0%, 10% and 70% respectively, indicating that the peptide-hyaluronate conjugate therefrom promotes wound healing.

**TABLE I**

| THE EFFECT OF TELIO-DERM™ ON SECOND DEGREE BURN HEALING* | | | | | | |
|---|---|---|---|---|---|---|
| TREATMENT | DAYS AFTER WOUNDING | | | | | |
| | 7 | 8 | 9 | 10 | 11 | 12 |
| Air Exposed | 0/15 (0%) | 0/15 (0%) | 0/15 (0%) | 6/15 (40%) | 7/14 (50%) | 14/14 (100%) |
| Hyaluronate | 0/10 (0%) | 1/10 (10%) | 1/10 (10%) | 10/10 (100%) | 10/10 (100%) | 10/10 (100%) |
| Peptide-conjugate | 2/10 (20%) | 4/10 (40%) | 9/10 (90%) | 9/9 (100%) | 9/9 (100%) | 9/9 (100%) |
| Tegaderm | 1/10 (10%) | 2/10 (20%) | 7/10 (70%) | 10/10 (100%) | 10/10 (100%) | 10/10 (100%) |

| | | | | | | |
|---|---|---|---|---|---|---|
| * Data is presented as number of wounds healed over number of wounds sampled. | | | | | | |
| () Percent of wounds epithelialized. | | | | | | |

Although the invention has been described with reference to the presently preferred embodiments, it should be understood that various modifications can be made by those skilled in the art without departing from the invention. Accordingly, the invention is set out in the following claims.

## Claims

1. A synthetic extracellular replacement matrix for wound healing comprising:
a. a synthetic peptide containing the amino acid sequence Y-Gly-Asp, wherein Y is R or dR, said peptide having cell attachment promoting activity; and
b. a biodegradable polymer bonded to said peptide without substantially reducing said affinity for cell binding, wherein said polymer is selected from the group consisting of hyaluronic acid, chondroitin sulfate, heparin, heparan sulfate, polylactate and polyglycolic acid;
wherein said matrix promotes cell attachment and migration therein and is not a solid support.

2. The matrix of claim 1, wherein said synthetic peptide further comprises at least one group of at least three identified amino acids in sequence selected from K, dK, R, dR, ornithine or D-ornithine.

3. The matrix of claims 1 or 2, wherein said peptide has the amino acid Lysine (K) at its C-terminus.

4. The matrix of claim 1, wherein said peptide is selected from the group consisting of:
G R G D S P K;
R R R R R R G D S P K;
G (dR) G D S P A S S K;
nMeG (dR) (dR) (dR) (dR) (dR) G G G (dR) G D S P A S S K;
G (dR) (dR) (dR) (dR) (dR) G G G (dR) G D S P A S S K; and
peptides containing any of the above sequences.

5. The matrix of claims 1 to 4 wherein, said biodegradable polymer comprises hyaluronic acid.

6. The matrix of claims 1 to 4, wherein said biodegradable polymer comprises chondroitin sulfate.

7. The matrix of claim 1, wherein said synthetic peptide is G (dR) (dR) (dR) (dR) (dR) G G G (dR) G D S P A S S K and the biodegradable polymer is hyaluronic acid.

8. The matrix of claim 1, wherein said synthetic peptide is G (dR) (dR) (dR) (dR) (dR) G G G (dR) G D S P A S S K and the biodegradable polymer is chondroitin sulfate.

9. The matrix of claims 1 to 8, further comprising a cross-linking agent.

10. The matrix of claim 9, wherein the cross-linking agent is selected from the group consisting of 1-ethyl-3-(3-dimethylaminopropyl) carbodiimide (EDC), dicyclohexylcarbodiimide, glutaraldehyde, cyanogen bromide and N-hydroxysuccinimide.

11. A method for preparing the synthetic extracellular replacement matrix according to claims 1 to 9 of an Y-Gly-Asp containing synthetic peptide, wherein Y is R or dR, and a biodegradable polymer selected from the group consisting of hyaluronate, chondroitin sulfate, heparin, heparan sulfate, polylactate and polyglycolic acid, comprising the step of bonding amino acid chains or termini of said peptide to a carboxyl group on said biodegradable polymer by means of a cross-linking/coupling agent.

12. The method of claim 11, wherein said cross-linking/coupling agent is selected from the group consisting of 1-ethyl-3-(3-dimethylaminopropyl) carbodiimide (EDC), dicyclohexylcarbodiimide, glutaraldehyde, cyanogen bromide and N-hydroxysuccinimide.

13. The method of claims 11 or 12, wherein said peptide is selected from the group consisting of:
G R G D S P K;
R R R R R R G D S P K;
G (dR) G D S P A S S K;
nMeG (dR) (dR) (dR) (dR) (dR) G G G (dR) G D S P A S S K;
G (dR) (dR) (dR) (dR) (dR) G G G (dR) G D S P A S S K; and
peptides containing any of the above sequences.

14. A synthetic peptide selected from the group consisting of nMe G (dR) (dR) (dR) (dR) (dR) G G G (dR) G D S P A S S K; and
G (dR) (dR) (dR) (dR) (dR) G G G (dR) G D S P A S S K.

15. Use of a synthetic extracellular replacement matrix according to claims 1 to 10 for the preparation of a pharmaceutical composition for the treatment of wounds.

16. Use according to claim 14, wherein said wounds are severe burns, skin graft donor sites, decubitus ulcers, diabetic ulcers, surgical incisions, or keloid-forming wounds.

17. Use according to claims 15 or 16, wherein said synthetic peptide is G (dR) (dR) (dR) (dR) (dR) G G G (dR) G D S P A S S K and the biodegradable polymer is hyaluronic acid.

18. Use according to claims 15 or 16, wherein said synthetic peptide is G (dR) (dR) (dR) (dR) (dR) G G G (dR) G D S P A S S K and the biodegradable polymer is chondroitin sulfate.

## Patentansprüche

1. Synthetische extrazelluläre Substitutionsmatrix für die Wundheilung, umfassend:
a. ein synthetisches Peptid, welches die Aminosäuresequenz Y-Gly-Asp enthält, in der Y R oder dR ist, wobei das Peptid eine die Zellanheftung fördernde Aktivität aufweist, und
b. ein biologisch abbaubares Polymer, welches an das Peptid gebunden ist, ohne die Affinität zur Zellbindung im wesentlichen zu reduzieren, wobei das Polymer ausgewählt ist aus der Gruppe bestehend aus Hyaluronsäure, Chondroitinsulfat, Heparin, Heparansulfat, Polylactat und Polyglycolsäure,
wobei die Matrix die Zellanheftung und die Migration in dieselbe fördert und nicht ein fester Träger ist.

2. Matrix nach Anspruch 1, bei der das synthetische Peptid ferner mindestens eine Gruppe von mindestens drei identifizierten aufeinanderfolgenden Aminosäuren ausgewählt aus K, dK, R, dR, Ornithin oder D-Ornithin umfaßt.

3. Matrix nach Anspruch 1 oder 2, bei der das Peptid an seinem C-Terminus die Aminosäure Lysin (K) aufweist.

4. Matrix nach Anspruch 1, bei der das Peptid ausgewählt ist aus der Gruppe bestehend aus:
G R G D S P K,
R R R R R R G D S P K,
G (dR) G D S P A S S K,
nMeG (dR) (dR) (dR) (dR) (dR) G G G (dR) G D S P A S S K,
G (dR) (dR) (dR) (dR) (dR) G G G (dR) G D S P A S S K, und
Peptiden, die irgendeine der obigen Sequenzen enthalten.

5. Matrix nach den Ansprüchen 1 bis 4, bei der das biologisch abbaubare Polymer Hyaluronsäure umfaßt.

6. Matrix nach den Ansprüchen 1 bis 4, bei der das biologisch abbaubare Polymer Chondroitinsulfat umfaßt.

7. Matrix nach Anspruch 1, bei der das synthetische Peptid G (dR) (dR) (dR) (dR) (dR) G G G (dR) G D S P A S S K ist und bei der das biologisch abbaubare Polymer Hyaluronsäure ist.

8. Matrix nach Anspruch 1, bei der das synthetische Peptid G (dR) (dR) (dR) (dR) (dR) G G G (dR) G D S P A S S K ist und bei der das biologisch abbaubare Polymer Chondroitinsulfat ist.

9. Matrix nach den Ansprüchen 1 bis 8, ferner umfassend ein Vernetzungsmittel.

10. Matrix nach Anspruch 9, bei dem das Vernetzungsmittel ausgewählt ist aus der Gruppe bestehend aus 1-Ethyl-3-(3-dimethylaminopropyl)carbodiimid (EDC), Dicyclohexylcarbodiimid, Glutaraldehyd, Bromcyan und N-Hydroxysuccinimid.

11. Verfahren zur Herstellung der synthetischen extrazellulären Substitutionsmatrix gemäß den Ansprüchen 1 bis 9 eines Y-Gly-Asp enthaltenden synthetischen Peptids, bei dem Y R oder dR ist, und einem biologisch abbaubaren Polymer ausgewählt aus der Gruppe bestehend aus Hyaluronat, Chondroitinsulfat, Heparin, Heparansulfat, Polylactat und Polyglycolsäure, umfassend die Schritte der Bindung von Aminosäureketten oder -termini des Peptids an eine Carboxylgruppe des biologisch abbaubaren Polymers mit Hilfe eines Vernetzungs-/Kopplungsmittels.

12. Verfahren nach Anspruch 11, bei dem das Vernetzungs/Kopplungsmittel ausgewählt wird aus der Gruppe bestehend aus 1-Ethyl-3-(3-dimethylaminopropyl)carbodiimid (EDC), Dicyclohexylcarbodiimid, Glutaraldehyd, Bromcyan und N-Hydroxysuccinimid.

13. Verfahren nach Anspruch 11 oder 12, bei dem das Peptid ausgewählt wird aus der Gruppe bestehend aus:
G R G D S P K,
R R R R R R G D S P K,
G (dR) G D S P A S S K,
nMeG (dR) (dR) (dR) (dR) (dR) G G G (dR) G D S P A S S K,
G (dR) (dR) (dR) (dR) (dR) G G G (dR) G D S P A S S K, und
Peptiden, die irgendeine der obigen Sequenzen enthalten.

14. Synthetisches Peptid ausgewählt aus der Gruppe bestehend aus
nMeG (dR) (dR) (dR) (dR) (dR) G G G (dR) G D S P A S S K, und
G (dR) (dR) (dR) (dR) (dR) G G G (dR) G D S P A S S K.

15. Verwendung einer synthetischen extrazellulären Substitutionsmatrix gemäß den Ansprüchen 1 bis 10 zur Herstellung einer pharmazeutischen Zusammensetzung zur Behandlung von Wunden.

16. Verwendung nach Anspruch 14, wobei die Wunden schwere Verbrennungen, Hauttransplantatspenderstellen, Dekubitalgeschwüre, Diabetesgeschwüre, operative Schnitte oder keloidbildende Wunden sind.

17. Verwendung nach Anspruch 15 oder 16, bei der das synthetische Peptid G (dR) (dR) (dR) (dR) (dR) G G G (dR) G D S P A S S K ist und bei der das biologisch abbaubare Polymer Hyaluronsäure ist.

18. Verwendung nach Anspruch 15 oder 16, bei der das synthetische Peptid G (dR) (dR) (dR) (dR) (dR) G G G (dR) G D S P A S S K ist und bei der das biologisch abbaubare Polymer Chondroitinsulfat ist.

## Revendications

1. Matrice synthétique de substitution extracellulaire pour la cicatrisation comprenant :
a) un peptide synthétique contenant la séquence d'acides aminés Y-Gly-Asp, où Y est R ou dR, ledit peptide ayant une activité favorisant la jonction cellulaire ; et
b) un polymère biodégradable relié au peptide sans en réduire de façon notable ladite affinité pour la liaison cellulaire, dans laquelle ledit polymère est choisi au sein du groupe constitué de l'acide hyaluronique, du sulfate de chondroitine, de l'héparine, de l'héparan sulfate, du polylactate et de l'acide polyglycolique ;
où ladite matrice favorise la jonction et la migration cellulaires et n'est pas un support solide.

2. Matrice selon la revendication 1, où ledit peptide synthétique comprend en outre au moins un groupe d'au moins trois acides aminés identifiés dans la séquence choisis parmi K, dK, R, dR, l'ornithine ou la D-ornithine.

3. Matrice selon la revendication 1 ou 2, dans laquelle ledit peptide a l'acide aminé Lysine (K) à son extrémité C-terminale.

4. Matrice selon la revendication 1, dans laquelle ledit peptide est choisi au sein du groupe constitué de :
G R G D S P K ;
R R R R R R G D S P K ;
G (dR) G D S P A S S K ;
nMeG (dR) (dR) (dR) (dR) (dR) G G G (dR) G D S P A S S K ;
G (dR) (dR) (dR) (dR) (dR) G G G (dR) G D S P A S S K ;
et des peptides contenant l'une quelconque des séquences ci-dessus.

5. Matrice selon les revendications 1 à 4, dans laquelle le polymère biodégradable comprend de l'acide hyaluronique.

6. Matrice selon les revendications 1 à 4, dans laquelle ledit polymère biodégradable comprend du sulfate de chondroitine.

7. Matrice selon la revendication 1, où ledit peptide synthétique est G (dR) (dR) (dR) (dR) (dR) G G G (dR) G D S P A S S K et le polymère biodégradable est l'acide hyaluronique.

8. Matrice selon la revendication 1, dans laquelle ledit peptide synthétique est G (dR) (dR) (dR) (dR) (dR) G G G (dR) G D S P A S S K et le polymère biodégradable est le sulfate de chondroitine.

9. Matrice selon les revendications 1 à 8, comprenant en outre un agent de réticulation.

10. Matrice selon la revendication 9, dans laquelle l'agent de réticulation est choisi parmi le groupe constitué du 1-éthyl-3-(3-diméthylaminopropyl) carbodiimide (EDC), le dicyclohexylcarbodiimide, le glutaraldéhyde, le bromure de cyanogène, et le N-hydroxysuccinimide.

11. Méthode pour la préparation d'une matrice synthétique de substitution extracellulaire selon les revendications 1 à 9, contenant un peptide synthétique de séquence Y-Gly-Asp, dans laquelle Y est R ou dR, et un polymère biodégradable choisi parmi le groupe consitutué du hyaluronate, du sulfate de chondroitine, de l'héparine, de l'héparan sulfate, du polylactate et de l'acide polyglycolique, comprenant l'étape de liaison des chaînes d'acides aminés ou des extrémités dudit peptide à un groupe carboxyl sur ledit polymère biodégradable au moyen d'un agent de réticulation/couplage.

12. Méthode selon la revendication 11, dans laquelle ledit agent de réticulation/couplage est choisi parmi le groupe constitué du 1-éthyl-3-(3-diméthylaminopropyl) carbodiimide (EDC), le dicyclohexylcarbodiimide, le glutaraldéhyde, le bromure de cyanogène et le N-hydroxy-succinimide.

13. Méthode selon la revendication 11 ou 12, dans laquelle ledit peptide est choisi parmi le groupe constitué de :
G R G D S P K ;
R R R R R R G D S P K ;
G (dR) G D S P A S S K ;
nMeG (dR) (dR) (dR) (dR) (dR) G G G (dR) G D S P A S S K ;
G (dR) (dR) (dR) (dR) (dR) G G G (dR) G D S P A S S K ;
et des peptides contenant l'une quelconque des séquences ci-dessus.

14. Peptide synthétique choisi parmi le groupe constitué du nMe G (dR) (dR) (dR) (dR) (dR) G G G (dR) G D S P A S S K; et
G (dR) (dR) (dR) (dR) (dR) G G G (dR) G D S P A S S K.

15. Utilisation d'une matrice synthétique de substitution extracellulaire selon les revendications 1 à 10 pour la préparation d'une composition pharmaceutique pour le traitement des plaies.

16. Utilisation selon la revendication 14, dans laquelle lesdites plaies sont des brûlures sévères, des sites donneurs de greffe de peau, des ulcérations du décubitus, des ulcères diabétiques, des incisions chirurgicales, ou des plaies formant des chéloïdes.

17. Utilisation selon les revendications 15 ou 16, dans laquelle ledit peptide synthétique est G (dR) (dR) (dR) (dR) (dR) G G G (dR) G D S P A S S K et le polymère biodégradable est l'acide hyaluronique.

18. Utilisation selon les revendications 15 ou 16, dans laquelle ledit peptide synthétique est G (dR) (dR) (dR) (dR) (dR) G G G (dR) G D S P A S S K et le polymère biodégradable est le sulfate de chondroitine.
